## Europäisches Patentamt

## European Patent Office

## Office européen des brevets

(19)

(11) Veröffentlichungsnummer: **0 063 621**
**B1**

(12) # EUROPÄISCHE PATENTSCHRIFT

(45) Veröffentlichungstag der Patentschrift:
**24.07.85**

(21) Anmeldenummer: **81103093.1**

(22) Anmeldetag: **24.04.81**

(51) Int. Cl.⁴: **C 05 F 3/00**

(54) **Aufbereitungsmittel für Abfallprodukte.**

(43) Veröffentlichungstag der Anmeldung:
**03.11.82 Patentblatt 82/44**

(45) Bekanntmachung des Hinweises auf die Patenterteilung:
**24.07.85 Patentblatt 85/30**

(84) Benannte Vertragsstaaten:
**AT BE CH DE FR GB IT LI LU NL SE**

(56) Entgegenhaltungen:
DE - A - 1 963 177
DE - A - 2 610 648
DE - B - 1 019 322
FR - A - 341 333
FR - A - 2 310 135
FR - A - 2 323 659
FR - A - 2 416 016
US - A - 3 286 691

**CHEMICAL ABSTRACTS, Band 79, Nr. 2, 16. Juli 1973, Seite 296 Zusammenfassung 9561x Columbus, Ohio, US C. ZAMFIR et al. "Influence of saponin upon the autopurification process of water"**
**CHEMICAL ABSTRACTS, Band 78, Nr. 25. Juni 1973, Seite 50, Zusammenfassung 155106y Columbus, Ohio, US S.A. VICHKANOVA et al. "Antimicrobial preparations of saponins"**
**CHEMICAL ABSTRACTS, Band 91, Nr. 21, 19. November 1979, Seite 565, Zusammenfassung 174096e Columbus,**

(73) Patentinhaber: **S.-K.-Pharma Dr. B. Schmittmann GmbH, Langenhorster Strasse 30, D-5620 Velbert (DE)**

(72) Erfinder: **Wustinger, Horst, Vogelweiderstrasse Nr. 94, A-4600 Wels (AT)**

(74) Vertreter: **Patentanwälte Dr. Solf & Zapf, Asamstrasse 8, D-8000 München 90 (DE)**

(56) Entgegenhaltungen: (Fortsetzung)
**Ohio, US D.A. WELCH et al. "Growth and composition of alfalfa fertilized in greenhouse trials with deproteinized juice from low and high saponin alfalfa and from oat herbage"**

## Beschreibung

Die Erfindung betrifft eine Aufbereitung von Abfallprodukten, wobei u.a. insbesondere Schadgas inhibiert wird, das bei der Lagerung oder Verwertung menschlicher und/oder tierischer Exkremente oder Abwässer oder dergleichen Abfallprodukte auftritt. Die Entwicklung derartiger unangenehm riechender und ggf. sogar schädlicher Gase soll zumindest vermindert und ggf. auch die Weiterverwendbarkeit der Abfallprodukte gefördert werden. Vornehmlich soll die Aufbereitung jedoch bei Fäkalien und tierischen Ausscheidungen in flüssiger Form (Gülle) erfolgen.

Im Zuge der Intensivierung und Rationalisierung der landwirtschaftlichen Nutztierhaltung wird in der Regel das sogenannte Flüssigmistverfahren insbesondere bei strohloser Aufstallung der Tiere angewendet. Dabei fällt die Gülle, ein Gemisch aus Kot, Harn und Wasser, an. Die Gülle schafft insbesondere drei zu bewältigende Probleme, nämlich
a) den Abbau der Schwimmdecke, die sich über der eigentlichen Gülle absetzt und die überwiegend aus Eiweiss- und Fettstoffen besteht,
b) die Verminderung der chemischen Aggressivität der Gülle,
c) die Verminderung der Geruchs- und Schadgasbildung ($NH_3$, $H_2S$).

Die Gülle beeinträchtigt insbesondere bei der Intensivtierhaltung, wenn Voll- und Teilspaltbödenställe benutzt werden, die Leistung und/oder Gesundheit der Tiere; denn die Tiere befinden sich meist fast unmittelbar über zumindest einer Teilmenge der Gülle. Geruch und Schadgase im Stall und in der Abluft stammen vom Tier selbst und aus den mit Exkrementen verschmutzten Boxen sowie gelagertem Kot und Harn in den Flüssigmistkanälen bei Teilspaltböden und im Güllekeller bei Vollspaltböden.

Der Flüssigmist – die Gülle – wird insbesondere durch anaerobe, untergeordnet jedoch auch durch aerobe Mikroorganismen, zersetzt. Es entstehen beim Abbau von organischen Substanzen (Tierkot und Eiweissverbindungen) neben unangenehm riechenden und schädlichen Gasen wie Ammoniak und Schwefelwasserstoff noch eine Reihe anderer chemischer Verbindungen, die auch Geruchsträger sind wie z.B. Aldehyde, Mercaptane, Amine, Methanindol, Skatol usw.

Insbesondere ist Ammoniak ein die Gesundheit schädigendes und die Leistung der Tiere beeinträchtigendes Schadgas. Die Temperatur in den Stallungen spielt dabei eine wesentliche Rolle, weil die optimale Leistung der Tiere durch sie beeinflusst wird. Je nach Grösse und Alter der Tiere liegt die günstigste Leistungstemperatur zwischen 15 und 24°C. Je höher die Temperatur ist, desto schneller laufen aber auch die mikrobiellen Zersetzungsvorgänge ab, und die Menge der dabei entstehenden Schadgase nimmt entsprechend zu.

Der Zersetzungsvorgang und die Schadgasbildung werden grösstenteils von den anaeroben Mikromismen vollzogen, da diese Mikromismen ohne Sauerstoff lebensfähig sind, so dass die sauerstoffarme Gülle ein sehr geeigneter Lebensraum für sie ist. Je weniger Sauerstoff in die Gülle gelangen kann, desto geringer ist die aerobe Bakterientätigkeit und desto intensiver ist die Zersetzung unter Schadgasbildung. Dazu kommt, dass bei starker Bevorzugung des anaeroben Wachstums durch das umgebende Milieu die Aerobier weitestgehend aus diesem verdrängt werden.

Die in den Tierexkrementen enthaltenen Feststoffe und Fettverbindungen bilden die sogenannte Schwimmdecke, welche innerhalb eines Mastdurchganges eine Dicke von 15 bis 20 cm erreicht. Aufgrund ihrer relativen Dichtheit können zwar die anfallenden Flüssigkeiten wie Harn und Wasser durchsickern, der relativ feste Kot der Tiere verbleibt jedoch an der Oberfläche, so dass sich die Schwimmdecke entsprechend der anfallenden Menge aufbaut. Die Schwimmdecke behindert aufgrund ihrer Dichtheit das Eindringen von Sauerstoff in die Gülle und unterbindet somit nahezu jegliche aerobe Mikromismentätigkeit. Sie ist ferner Brutstätte unzähliger Bakterienstämme.

Die sich unter der Schwimmdecke bildenden zahlreichen, meist sehr übelriechenden Gase, welche bei der Zersetzung der organischen Stoffe zudem entstehen, werden von den in der Gülle befindlichen, sehr gross molekularen Geruchsträgern aufgenommen und eingebunden, so dass die Geruchsintensität der Gülle immer mehr zunimmt und ihre volle Intensität bei der Freisetzung der Gase z.B. durch Einwirkung von Luftsauerstoff, beispielsweise zum Zeitpunkt der Ausbringung auf landwirtschaftliche Böden, zur Wirkung kommt. Zudem bauen sich in der Gülle noch eine Reihe von schädlichen Stickstoffverbindungen auf, welche das sogenannte «Verbrennen» des landwirtschaftlichen Bodens bewirken, wenn die Gülle unmittelbar als Dünger auf das Feld gebracht wird (Kopfdüngung).

Umfangreiche Forschungen und Versuche haben aufgezeigt, welche wirtschaftliche Bedeutung den Schadgasen in bezug auf die Gesundheit und Leistung der Tiere zuzurechnen ist. Es wurden die Auswirkungen dieser Schadgase auf Gewichtszunahme, Futterverwertung und Ausfallquoten bei Schweinen durch zahlreiche Versuche festgestellt, wobei nach DIN 18910 zulässige Werte für $NH_3$ und $H_2S$ zugrundegelegt wurden.

Ähnliche Probleme treten bei der Abwasserbewältigung, z.B. in Kläranlagen, Teichen oder Flüssen, auf. Hinzu kommt hier noch das Sedimentationsproblem bei Schwebstoffen, die schädlichen Braunalgen und die Faulschlammbildung. Für letztere ist im wesentlichen ebenfalls die anaerobe Mikroorganismentätigkeit verantwortlich.

Auch bei der Kompostierung von Mist, z.B. von Schweine-, Hühner- oder Schafsmist, entstehen durch die anaerobe Mikroorganismentätigkeit für die Düngung schädliche Stickstoffverbindungen und unangenehm riechende Gase. Ausserdem dauert die Verrottung des Mists sehr lange.

Eine Lösung für die insbesondere im Zusammenhang mit der Gülle aufgezeigten komplexen Probleme ist aus dem Stand der Technik nicht bekannt.

In den meisten Fällen behilft man sich mit einer starken Belüftung des Stalles bzw. einem Absaugen der Schadgase. Diese Verfahrensweise ist nicht effektiv genug und bedingt Zug im Stall, der Erkältungskrankheiten der Tiere verursachen kann.

Andere Verfahren sehen den Zusatz von Enzymen oder Chemikalien zur Gülle vor. Die Chemikalien sollen entweder den pH-Wert im sauren Bereich stabilisieren oder die Schadgase binden oder deren Bildung verzögern oder forcieren oder die Geruchsbelästigung überdecken durch eine angenehmere Geruchsentwicklung (Kaschierung). Die Wirkung derartiger Chemikalien ist jedoch ungenügend. Vielfach bedingen die Chemikalien zudem den Nachteil, dass die Gülle wegen der aus den Chemikalien stammenden agrikulturschädlichen Ionen nicht auf landwirtschaftliche Nutzflächen ausgebracht werden kann.

Darüber hinaus ist bekannt, die Flüssigkeit der Gülle durch Adsorbtivstoffe zu binden und durch die Adsorbtion feststoffartige Produkte zu bilden. In geringem Umfange soll dabei auch die Geruchsbelästigung gemindert werden. Nachteilig ist hierbei zum einen, dass die Zuschlagstoffe in bedeutenden Mengen verwendet werden müssen und dass sie in der Regel teuer sind. In diesem Zusammenhang ist auch bekannt geworden, die Gülle mit gebranntem Kalk zu trocknen. Der gebrannte Kalk bindet die Gülle adsorbtiv, wobei Wärme entwickelt wird, die zur Verdampfung der Flüssigkeit führen soll. Gebrannter Kalk ist jedoch sehr teuer, und ausserdem kann die Geruchsbelästigung bzw. Schadgasbildung nicht unterbunden werden. Ausserdem entsteht durch die stark desinfizierende Eigenschaft des gebrannten Kalkes sogenanntes totes Material, in dem bakterielles Leben weitestgehend abgetötet ist.

Bekannt ist ferner, dass sich die Schadgase vornehmlich bei der anaeroben Zersetzung von Eiweissstoffen bilden. Man hat daher versucht, aerobe Verhältnisse zu schaffen, indem man Luft mechanisch in sogenannte Oxidationsgräben oder oberirdische Behälter einträgt. Dieses Verfahren erfordert einen erheblichen Aufwand und hohe laufende Betriebskosten. Die dafür erforderlichen Anlagen lassen sich zudem baulich nicht überall installieren. Ausserdem lösen diese Anlagen nicht das Problem beim Transport und beim Ausbringen der Gülle.

Aus dem Dokument «Chemical Abstracts», Bd. 79, Nr. 2, 16. Juli 1973, Seite 296, Zusammenfassung 9561x Columbus, Ohio, US. C. Zamfir et al. «Influence of Saponin upon the autopurification process of water» ist die Behandlung von Abwasser bekannt, das bei der Herstellung von Zucker aus Zuckerrüben anfällt. Zuckerrüben enthalten in beachtlichen Mengen Saponin. Dieses Saponin gelangt bei der Raffinade in das Abwasser. Das Abwasser kann aus diesem Grund nicht ohne weiteres in Flüsse oder dgl. abgeleitet werden, da Saponin toxisch wirkt. Die maximal mögliche Konzentration des Saponins im Abwasser wird nach dem Bericht festgestellt und die Toxizität ermittelt. Es wird vorgeschlagen, das Abwasser zu verdünnen, so dass der Saponingehalt nicht 0,2 mg/ml übersteigt. Eine Verwendung von Saponin bei Abfallprodukten geht aus dem Dokument nicht hervor.

Aufgabe der Erfindung ist, mit einem Aufbereitungsmittel in Abfallprodukten, insbesondere im Flüssigmist, in Abwässern, bei der Kompostierung oder in dergleichen Abfallprodukten, die nachteiligen Wirkungen der anaeroben Vorgänge weitestgehend zu unterdrücken.

Diese Aufgabe wird erfindungsgemäss durch die Verwendung eines neben Wasser Saponin oder ein Saponin in wirksamer Form enthaltenden Produkts als Aufbereitungsmittel für Abfallprodukte gelöst. Das Saponin in wirksamer Form enthaltene Produkt kann ein Zwischenprodukt zur Herstellung handelsüblicher Saponinextraktprodukte sein. Vorzugsweise können monodesmosidische Saponine aus z.B. Quillajarinde, Seifenwurzeln, Spinat, Guyak, Yuka, Rosskastanien, Rüben verwendet werden.

Die Saponine sind eine Gruppe von Glykosiden, die sich durch eine Reihe von gemeinsamen Merkmalen auszeichnen. Es sind pflanzliche Stoffe, die in Wasser gelöst die Oberflächenspannung beeinflussen und – ähnlich wie Seife beim Schütteln – einen haltbaren Schaum liefern. Sie bestehen aus Kohlenstoff, Wasserstoff und Sauerstoff und weisen ein hohes Molekulargewicht auf. Als Inhaltsstoffe vieler Pflanzen sind die Saponine in der Natur weit verbreitet. Ihre Gewinnung erfolgt durch auf die Droge abgestimmte Extraktions- und Aufbereitungsverfahren. Der Abschluss des Verarbeitungsprozesses ist im allgemeinen die Überführung der Extrakte in die Pulverform. In dieser Form gelangen die Saponine in den Handel. In Fällen, in denen es nicht möglich ist, die Saponinextrakte in die Pulverform zu überführen, werden diese in einer stark konzentrierten, flüssigen, meist konservierten Form angeboten. Die aus verschiedenen saponinhaltigen Drogen gewonnen Saponine unterscheiden sich mehr oder weniger untereinander. Eine chemische Unterscheidung wird anhand ihres zuckerfreien Grundgerüstes, des sogenannten Aglykons oder auch Sapogenins, getroffen. Die zwei grossen dabei zu unterscheidenden Gruppen sind:
1) Sapogenine mit Triterpenstruktur (Oleanolsäure-Grundgerüst)
2) Spirostanole (Steranskelett-Digitogenin)

Saponine sind in Heilmitteln und Waschmitteln hoch begehrt. Heute hat sich die Anwendung von Saponinen mehr auf andere Gebiete verlagert. Beispielsweise wird Saponin aus Quillaja-Saponaria gewonnen. Es ist ein auf besonderem Wege gereinigter und in Pulverform überführter, hochwertiger, in Wasser in jedem Verhältnis löslicher Drogenextrakt. Das im Saponinanteil benannte Hauptsaponin, Quillaja-Saponin, ist ein Glykosid, dessen Aglykon als Triterpenoid der β-Amyrin-Oleanolsäure-Gruppe angehört. Die bei der Hydrolyse abgespaltenen Zucker sind bisher im einzelnen nicht bekannt. Quillaja-Saponin wirkt hämolytisch. Das Pulver reizt beim Einatmen die Schleimhäute. Es wird hauptsächlich zur Herstellung von Filmemulsionen verwendet und ist frei von Härtebildnern und Schwermetallen. Als Pulver ist es weiss bis fast weiss und ausser in Wasser in Dioxan löslich. Die 5%-ige wässrige Lösung hat einen pH-Wert zwischen 4 und 5. Die Haltbarkeit der Saponinlösung beträgt im offenen Gefäss und bei einer Aussentemperatur von 15°C durchschnittlich drei bis fünf Tage. Die Haltbarkeit verlängert sich bei Temperaturen unter 10°C und wird geringer oberhalb 20°C. Die Haltbarkeit der Lösung kann durch Aufbewahren in Kühlräumen, Aufkochen und Aufbewahren in geschlossenen Behältern, Zugabe von Äthylalkohol bis zu 18% und Zugabe von Konservierungsmitteln verlängert werden. Die Einarbeitung von Konservierungsmitteln verändert jedoch die natürliche Zusammensetzung der Lösung erheblich.

Gypsophilasaponin wird aus Radix-Saponaria in der Regel ebenfalls als in Pulverform überführter Drogenextrakt gewonnen. Es ist – ebenso wie das Quillaja-Saponin – zur Herstellung von Film- und Fotopapieren verwendbar und weist dabei etwa die gleichen Eigenschaften auf. Darüber hinaus findet es hauptsächlich Verwendung als Netzmittel, in der kosmetischen, galvanischen und Getränke-Industrie und wird zur Förderung der Aufnahme von Arzneien in der pharmazeutischen Industrie eingesetzt.

Weitere Einsatzgebiete der Saponine sind in Abhängigkeit vom Reinheitsgrad die Lebensmittelindustrie, Lack- und Farbindustrie, der Reprobereich, die Getränkeindustrie und die Verwendung zu Reinigungszwecken. In den meisten Fällen wird Saponin eingesetzt, um die Oberflächenspannung von Flüssigkeiten oder dergleichen zu beeinflussen.

Handelsübliche Saponinprodukte sind Saponinextraktlösungen, Saponinpulver und Granulate, die in der Regel einen Reinsaponingehalt von etwa 5 bis 30 Gew.-% aufweisen.

Nach einer Weiterbildung der Erfindung werden vorzugsweise Saponinextraktprodukte verwendet. Aus diesen Produkten wird eine verdünnte Lösung in Wasser mit einem Reinsaponingehalt von etwa 10 bis 20 ppm hergestellt. Dieses Aufbereitungsmittel wird z.B. in den Flüssigmist eingebracht oder aber oberflächlich verteilt, z.B. aufgesprüht oder aufgedüst oder dergleichen.

Die erfindungsgemässe Verwendung von Saponin enthaltenden Produkten als Aufbereitungsmittel verändert offenbar die Reaktionsbedingungen im Abfallprodukt zugunsten des aeroben Bakterienwachstums derart, dass die aeroben Vorgänge schneller ablaufen als die anaeroben oder die anaeroben Vorgänge weitestgehend unterdrückt werden. Es ist derzeit nicht bekannt, worauf die unerwartete günstige Eigenschaft der erfindungsgemässen Verwendung zurückzuführen ist. Dabei lag es keineswegs nahe, Saponinprodukte zur Lösung der erfindungsgemässen Aufgabe einzusetzen, wenn man berücksichtigt, dass die Saponine in Lösungen durch Mikroorganismen rasch zersetzt werden und daher befürchtet werden musste, dass das Saponin zumindest in den zugesetzten geringen Konzentrationen völlig unwirksam bleibt. Unter anderem ist zwar in der Literatur auch berichtet worden, dass Saponin auf die meisten Bakterien wachstumsfördernd wirkt. Überraschend ist jedoch, dass im Falle der Erfindung diese Wirkung offenbar selektiv zugunsten des Wachstums der aeroben Bakterien abläuft. Würde das Wachstum der aeroben und anaeroben Bakterien gleichermassen gefördet, was der Fachmann hätte erwarten müssen und weshalb er Saponin bisher wohl auch nicht eingesetzt hat, so hätte sich lediglich eine Beschleunigung der bekannten Vorgänge mit den beschriebenen Nachteilen ergeben. Für den Durchschnittsfachmann ergibt sich aus dem Stand der Technik somit weder ein Hinweis, der die Verwendung von Saponin für die Zwecke der Erfindung nahegelegt hätte, noch kann angenommen werden, dass die Auswahl der Saponinprodukte aus einer Unzahl chemischer Produkte für den Durchschnittsfachmann ohne weiteres getroffen werden konnte.

Es wird – wie oben bereits erwähnt – angenommen, dass die hochverdünnte Saponinlösung das aerobe Bakterienwachstum so stark beschleunigt, dass das anaerobe Bakterienwachstum, das unter normalen Bedingungen sehr viel schneller abläuft, zurückgedrängt wird oder erst einsetzt, wenn das aerobe Bakterienwachstum fast abgeschlossen ist, so dass für das anaerobe Bakterienwachstum kein Nährboden mehr vorhanden ist.

Neben der Förderung des selektiven Bakterienwachstums initiiert die Verwendung von Saponin sehr wahrscheinlich auch einen Gärungsprozess, bei dem bekanntlich fermentative und enzymatische bzw. sogenannte biokatalytische Vorgänge ablaufen.

Im vorliegenden Fall laufen die meisten Reaktionen bezüglich der im Abfallprodukt enthaltenen Proteine wohl nicht bis zur $NH_3$-Bildung ab, sondern enden ggf. mit der Bildung von Aminosäuren. Dadurch können intakte Moleküle mit chemisch gebundenem Stickstoff erhalten bleiben. Das Aufbereitungsprodukt weist somit einen hohen Stickstoffgehalt auf, der den Wert als Düngemittel erheblich steigert.

Besonders wirksam ist nach einer Weiterbildung der Erfindung eine Verwendung, bei der die verdünnte Saponinlösung einen Stoff enthält, der als Nährboden für Bakterien dient und/oder als Keimbildner für das Wachstum der Bakterien wirkt. Vorzugsweise sollten diese Stoffe das Wachstum der aeroben Bakterien fördern.

In überraschender Weise hat sich in diesem Zusammenhang ergeben, dass als Nährstoffe Produkte verwendbar sind, die bekanntermassen als Nährstoff sowohl für aerobe als auch für anaerobe Bakterien gelten; dennoch wird überwiegend nur das Wachstum der aeroben Bakterien durch die Nährstoffe gefördert, wenn die Nährstofe mit den Saponinen zusammenwirken. Diese Tatsache kann lediglich mit einem synergistischen Effekt begründet werden, der sich aus der gegenseitigen Beeinflussung der beiden Stoffe ergibt. Nach einer Weiterbildung der Erfindung beschleunigt in besonderem Masse Melasse das aerobe Bakterienwachstum. Verwendbar als Nährstoffe sind z.B. aber auch Rohrzucker oder Lösungen anderer Zuckerprodukte. Ferner können z.B. Gelatine, Stärke oder Malzprodukte zugesetzt werden. Alle diese Stoffe bilden im Zusammenwirken mit der Saponinlösung eine Brutstätte für die aeroben Bakterien.

Zweckmässig ist, dem verdünnten, Saponin und mindestens einen Bakteriennährstoff enthaltenden Aufbereitungsmittel ein wasserlösliches Hydrokolloid zuzusetzen, das insbesondere als Quell- und/oder Verdickungs- und/oder Thixotropierungsmittel wirkt. Dieses Mittel kann zweckmässigerweise ein Aktivator für den Nährstoff sein. Bevorzugt werden in diesem Falle wasserlösliche Alginate, z.B. Natriumalginat oder pulverisierte, getrocknete Algen. Nach einer besonderen Ausführungsform der Erfindung wird ein Xanthan zugesetzt. Vorzugsweise wird Kelzan D verwendet.

Zweckmässig ist ferner, wenn nach einer weiteren Ausführungsform der Erfindung das verwendete, insbesondere Bakteriennährstoff enthaltende Aufbereitungsmittel ein an sich bekanntes Konservierungsmittel enthält, das verhindert, dass in dem Wasser enthaltenden Aufbereitungsmittel mikroorganische Prozesse ablaufen, bevor das Aufbereitungsmittel seiner bestimmungsgemässen Verwendung zugeführt worden ist. Vorzugsweise werden die bekannten zugelassenen Konservierungsmittel wie Sorbit, Sorbinsäure, Sorbate, Benzoesäure oder Tylose verwendet.

Nach einer Weiterbildung der Erfindung wird ein Konzentrat als Aufbereitungsmittel verwendet, das vorzugsweise in der folgenden Zusammensetzung zubereitet und in dieser Zubereitung gehandelt wird:

6 bis 20 Gew.-% Saponinprodukt mit einem Reinsaponingehalt von 20 bis 30 Gew.-%
40 bis 50 Gew.-% Melasse
1 bis 6 Gew.-% wasserlösliches Hydrokolloid
0,1 bis 6 Gew.-% Konservierungsmittel
Rest Wasser

In dieser Zusammensetzung werden die fermentativen und enzymatischen Vorgänge besonders begünstigt.

Diesem Konzentrat wird kurz vor der Verwendung eine weitere Wassermenge zugesetzt, so dass das Konzentrat in Mengen von 3 bis 55 ppm im Wasser vorliegt. Dieses Produkt wird dann bestimmungsgemäss eingesetzt.

Neben der beschriebenen unerwarteten Wirkung des Saponins auf das aerobe Bakterienwachstum bedingt dessen Verwendung zudem den besonderen Vorteil, dass bekanntlich die Oberflächenspannung von insbesondere Wasser erheblich herabgesetzt und die Benetzbarkeit von Feststoffen erheblich erhöht wird. Diese Eigenschaft unterstützt insbesondere die Wirkung der anderen Zusatzstoffe bzw. deren Verteilung in der Gülle sowie die Wirkung der Aufbereitungsmittel für die Verrottung der Feststoffe bei der Kompostierung.

Die Erfindung zeigt im Vergleich zum Stand der Technik somit einen völlig anderen Weg zur Lösung der genannten Probleme auf, nämlich das aerobe gegenüber dem anaeroben Bakterienwachstum zu fördern, indem Stoffe zugesetzt werden, die vornehmlich auf das aerobe Bakterienwachstum positiv einwirken und/oder einen Gärungsprozess hervorrufen. Es handelt sich dabei im wesentlichen um Naturprodukte bzw. durch Fermentation oder Extraktion aus organischen Naturprodukten, vornehmlich Pflanzen, gewonnene Stoffe (Saponin, Melasse, Alginate).

Die überraschend hervorragende Wirkung der Verwendung von Saponinprodukten als Aufbereitungsmittel für Gülle mit der folgenden bevorzugten Zusamensetzung wird nunmehr beispielhaft verdeutlicht:

8 Gew.-% Saponinextraktprodukt mit einer Konzentration von 30% Reinsaponin
45 Gew.-% Melasse
2 Gew.-% Kelzan D
2 Gew.-% Konservierungsmittel (Sorbinsäure)
Rest Wasser

Diesem Konzentrat wird Wasser zugegeben, so dass das Konzentrat mit 50 ppm im Wasser enthalten war. Diese Verdünnung wird mit etwa 0,1 l/m² zu behandelnder Oberfläche aufgebracht. Die folgenden Beispiele verdeutlichen, wie insbesondere die Schadgasbildung und der Abbau der Schwimmdecke in der Gülle vermindert wird und welche Vorteile die verminderte Schadgasbildung bei der Nutztierhaltung bedingt.

Beispiel 1
Technische Werte des Versuchsstalles I : Schweinestall

Teilspaltenbodenstall
| | |
|---|---|
| Güllekanal | 75% gefüllt |
| Schwimmdecke | 5–7 cm Dicke |
| Stalluft | übel riechend – ätzend |
| Verunreinigung | grosse Mengen Fäkalien seitlich der Spalten und des Kanals |
| Lüftung | Friscomat |

| | |
|---|---|
| Fläche | 80 m² |
| Anzahl der Tiere | 60 Schweine |
| ⌀ Gewicht der Tiere | 60 kg |
| Temperatur | 21°C |
| Luftbewegung | ca. 0,1 m/sec. |
| Luftfeuchtigkeit | ca. 75% |

Schadgaskonzentration vor der Behandlung:
Ammoniak NH$_3$      30 ppm

Behandlung durch die erfindungsgemässe Verwendung von Saponinprodukten als Aufbereitungsmittel:
Schadgasmessung nach 6 Tagen ergab folgende Werte:
Ammoniak NH$_3$      20 ppm ≙ 35% Reduzierung

Nochmalige Behandlung mit gleicher Dosis am Tage der Schadgasmessung.

Kontrollmessung nach weiteren 3 Tagen – insgesamt neunter Tag nach Erstbehandlung – ergab folgende Werte:
Ammoniak NH$_3$      11 ppm ≙ 45% Reduzierung

Schadgasreduzierung nach 9 Tagen somit 63,5%

| | |
|---|---|
| Schwimmdecke | Sie war nach 9 Tagen völlig abgebaut; Güllegeruch wesentlich vermindert. |
| Stalluft | Geruchsintensität stark vermindert – lediglich Tiergeruch wahrnehmbar. |

Beispiel 2
Technische Werte des Versuchsstalles II : Ferkelstall

| | |
|---|---|
| Teilspaltenbodenstall | |
| Güllebehälter | innenliegend 75% gefüllt |
| Schwimmdecke | 5–7 cm Dicke |
| Stalluft | übel riechend – stark ätzend |
| Verunreinigung | Fäkalien befanden sich in grösseren Mengen in den Ecken und auf den Spalten |
| Lüftung | ein kippbares Fenster |
| Fläche | 25 m² |
| Anzahl der Tiere | 60 Ferkel |
| ⌀ Gewicht der Tiere | 25 kg |

Schadgaskonzentrationen vor der Behandlung:
Ammoniak NH$_3$      15 ppm
Behandlung durch die erfindungsgemässe Verwendung von Saponinprodukten als Aufbereitungsmittel:

Schadgasmessungen nach 6 Tagen ergaben folgende Werte :

Ammoniak NH$_3$      5 ppm ≙ 66% Reduzierung

Nochmalige Behandlung mit gleicher Dosis am Tage der Schadgasmessung

Kontrollmessungen nach weiteren 3 Tagen – insgesamt neunter Tag nach Erstbehandlung – ergaben folgende Werte:
Ammoniak NH$_3$      3 ppm ≙ 40% Reduzierung

Schadgasreduzierung nach 9 Tagen somit ca. 80%

| | |
|---|---|
| Schwimmdecke | Sie war nach 6 Tagen völlig abgebaut; Güllegeruch wesentlich vermindert |
| Stalluft | Geruchsintensität stark vermindert – lediglich Tiergeruch wahrnehmbar |

Beispiel 3
Technische Werte des Versuchsstalles III:

| | |
|---|---|
| Umlauf – Tieflaufstall | |
| Mistmenge | von 2 Mastdurchgängen, teilweise Stroheinstreuung |
| Lüftung | keine mechanische, nur zwei gegenüberliegende Fenster, 3–5 cm kippbar |
| Holzdecke | |
| Fläche | 30 m² |
| Anzahl der Tiere | 23 |
| ⌀ Gewicht der Tiere | 100 kg |
| Stalluft | intensiver, üblicher Geruch – ätzend sehr geruchsintensiv |

Schadgaskonzentration vor der Behandlung:
Ammoniak NH$_3$       20 ppm

Behandlung durch die erfindungsgemässe Verwendung von Saponinprodukten als Aufbereitungsmittel:
Schadgasmessung nach 9 Tagen ergab folgende Werte:
Ammoniak NH$_3$       4 ppm ≙ 80% Reduzierung

Nochmalige Behandlung am Tage der Schadgasmessung mit gleicher Dosis

Kontrollmessung nach 3 Tagen – insgesamt zwölfter Tag nach der Erstbehandlung – ergab folgende Werte:
Ammoniak NH$_3$       3 ppm

| | |
|---|---|
| Mist | Er wurde am dreizehnten Tag nach der Erstbehandlung ausgebracht und war bereits nahezu geruchsfrei. Geruchswahrnehmung erst in unmittelbarer Nähe des Mistes. |
| Stalluft | Geruchsintensität stark reduziert – lediglich Tiergeruch wahrnehmbar. |

Beispiel 4
Technische Werte des Versuchsstalles IV:

| | |
|---|---|
| Vollspaltenbodenstall | |
| Güllekeller 175 m² | 75% gefüllt – Gülle 6 Monate in Deponie |
| Schwimmdecke | 25–30 cm Dicke, geschlossen |
| Stalluft | übelriechend – ätzend |
| Verunreinigungen | keine |
| Lüftung | Friskomat |
| Fläche | 80 m² |
| Anzahl der Tiere | 103 Schweine |
| ⌀ Gewicht der Tiere | 100 kg |
| Temperatur | 21 bis 23°C |
| Luftbewegung | 0,1 m/sec. |
| Luftfeuchte | ca. 65% |
| Kontrollstall | gleiche Werte |

Schadgaskonzentrationen vor der Behandlung:

| | Versuchsstall | Kontrollstall |
|---|---|---|
| Ammoniak NH$_3$ | 45 ppm | 45 ppm |
| Schwefelwasserstoff H$_2$S | 4 ppm | 4 ppm |

Behandlung durch die erfindungsgemässe Verwendung von Saponinprodukten als Aufbereitungsmittel:
Schadgasmessungen nach 9 Tagen ergaben folgende Werte:

| | Versuchsstall | Kontrollstall |
|---|---|---|
| Ammoniak NH$_3$ | 30 ppm | unveränderte Werte |
| Schwefelwasserstoff H$_2$S | 2 ppm | |

Nochmalige Behandlung am Tage der Schadgasmessung mit gleicher Dosis

Kontrollmessungen nach weiteren 3 Tagen – insgesamt zwölfter Tag nach der Erstbehandlung – ergaben folgende Werte:

| | Versuchsstall | Kontrollstall |
|---|---|---|
| Ammoniak NH$_3$ | 25 ppm | unveränderte Werte |
| Schwefelwasserstoff H$_2$S | 0 ppm | |

| | |
|---|---|
| Schwimmdecke | Sie war bereits wesentlich homogener; Teilbereiche (ca. 3–4 m²) waren bereits schwimmdeckenfrei. Am fünfzehnten Tage nach der Erstbehandlung wurde die Schwimmdecke aufgemixt, wozu ein Zeitraum von 30 Minuten benötigt wurde. |

Schadgasmessung 1 Stunde nach Aufmixung ergab:

| | |
|---|---|
| Ammoniak NH$_3$ | 20 ppm |
| Schwefelwasserstoff H$_2$S | 0 ppm |

Geruchsverminderung von Gülle und Stalluft war festzustellen.

Als wichtige Nebenwirkung der erfindungsgemässen Verwendung von Saponinprodukten als Aufbereitungsmittel ergab sich, dass im Kontrollstall die Gewichtszunahme der Tiere etwa 16% schlechter war als normal, während im Versuchsstall die schlechtere Gewichtszunahme bei einer 50%igen Schadgasreduzierung nur noch 8% betrug. Als weitere besondere Vorteile sind zu nennen eine prozentmässige Verbesserung der Futterverwertung, Verkürzung der Mastdauer durch eine höhere Gewichtszunahme pro Tag, Reduzierung der durch Schadgaseinwirkung bedingten Ausfallquoten sowie relativ geringe Kosten der Gülleaufbereitung und Reduzierung der Umweltbelastung durch Schadgasemission.

Die Beispiele zeigen, dass die Freisetzung von Ammoniak und Schwefelwasserstoff stark reduziert werden und somit die Schadgasbildung weitestgehend unterbunden wird. Das Saponin bewirkt zudem eine rasche Dispergierung der Schwebstoffe und der fettähnlichen Verbindungen. Es wird die Bildung von Schwimmdecken wesentlich verhindert, und es werden bestehende Schwimmdecken abgebaut. Dadurch wird die Oberflächenspannung der Gülle wesentlich vermindert, wodurch auch der Luftsauerstoff besser durch die Gülleoberfläche eindringen und den aeroben Bakterienwuchs noch wesentlich mehr unterstützen und aktivieren kann. Die Gülle wird zu einem gleichmässigen, nahezu homogenen Gemisch aufbereitet, das gleichmässig auf landwirtschaftliche Flächen aufgebracht werden kann. Durch die Homogenität der Gülle wird ausserdem die sogenannte Fleckdüngung, die bei inhomogenen Düngemitteln auftritt, ausgeschaltet.

Ferner fördert die erfindungsgemässe Verwendung von Saponinprodukten als Aufbereitungsmittel die Verrottung der Feststoffe bei der Kompostierung. Durch die Zerlegung von organischen Substanzen wird der Schweine-, Hühneroder Schafmist einer besseren Verrottung zugeführt und die schädlichen Stickstoffverbindungen sehr rasch aufgebaut. Neubildungen werden unterbunden. Der Schweine-, Hühner- oder Schafmist kann somit schon nach kurzer Zeit als Düngemittel verwendet werden. Bei der Kompostierung soll das Aufbereitungsmittel in einer Konzentration von vorzugsweise 70 bis 80 ppm, vorzugsweise 75 ppm, verwendet werden. Dieses Konzentrat wird auf die Mistlagen versprüht. Dabei sollte der Mist in Schichten von 20 bis 30 cm Dicke aufgetragen und die jeweilige Schicht mit dem Aufbereitungsmittel besprüht werden.

Bei der erfindungsgemässen Verwendung von Saponinprodukten als Aufbereitungsmittel hat sich in der Wasserwirtschaft gezeigt, dass die in Kläranlagen normalerweise aufgrund der anaeroben Bakterientätigkeit beim Zersetzen von pflanzlichen und organischen Substanzen sehr intensiv auftretende Geruchsbildung vermieden werden kann. Ferner werden die in den Abwässern enthaltenen Fette und fetthaltigen Substanzen sowie die Schwimmdecke abgebaut. In Teichen wird die Geruchsbildung ebenfalls unterbunden und die Braunalgenbildung verhindert. Diese Wirkung beruht auf der Begrenzung der anaeroben Bakterientätigkeit und der Förderung eines Gärungsprozesses.

Die erfindungsgemässe Verwendung von Saponinprodukten als Aufbereitungsmittel unterbindet nicht nur die unerwünschten Reaktionsabläufe in Abfallprodukten, sondern es kann dabei auch – wie beschrieben – wertvolle Düngemittel produzieren. Darüber hinaus ist es aber möglich, Abfallprodukte, die von Hause aus noch hohe Nährstoffe enthalten, so aufzubereiten, dass sie als zumindest Beimengen zu Futtermitteln zugegeben werden können.

Bekannt ist beispielsweise, dass Hühnerkot ein Produkt ist, das noch sehr viele wertvolle, unverbrauchte Nährstoffe enthält, weil die Verdauung des Huhns nicht in der Lage ist, die Nährstoffe während der Verdauungsdauer aus dem Futtermittel herauszuholen. Hühnerkot ist deshalb bevorzugt zu Düngemitteln verarbeitet worden (DE-Auslegeschrift 1 019 322, DE-Offenlegungsschriften 1 963 177, 2 539 930 und 2 610 648).

Durch die Aufbereitung des Geflügelkots, insbesondere des Hühnerkots, durch die erfindungsgemässe Verwendung von Saponinprodukten und ggf. durch den Zusatz weiterer, insbesondere adsorbtiv wirkender Stoffe (Diatomeenerde, Kieselgut oder pyrogene Kieselsäure), kann ein Produkte erzeugt werden, das zumindest als Beimengung zum Futter verwendet werden kann. Dieses Recycling von Abfallprodukten kann bis zur optimalen Ausbeutung der Nährstoffe wiederholt werden, so dass die Tierhaltung auch insoweit optimiert werden kann. Das Aufbereitungsmittel leistet dabei durch die Förderung des aeroben Bakterienwachstums und/oder der Gärung die beschriebenen Reaktionen wie insbesondere die Schadgasverminderung und ggf. sogar eine Entwicklung zusätzlicher wertvoller Nahrungsmittelstoffe aus den Kotbestandteilen, die von den Tieren leichter verdaut werden können.

In gleicher Weise können aber auch andere landwirtschaftliche Nebenprodukte, die hohe Nährstoffanteile aufweisen, wie z.B. Stroh, insbesondere Maisstroh oder Reisschalen oder dergleichen, durch die erfindungsgemässe Verwendung von Saponinprodukten als Aufbereitungsmittel kompostiert werden, woraus z.T. wertvolle Futtermittel resultieren. Die Kompostierung mit dem Aufbereitungsmittel erfordert sehr viel weniger Zeit als die Kompostierung mit üblichen Mitteln, weil im erfindungsgemässen Fall das Saponin nicht nur das aerobe Bakterienwachstum und die Benetzbarkeit der Feststoffe, sondern auch die Permeabilität von Feststoffhülsen erhöht, so dass der Verrottungsprozess ganz erheblich beschleunigt wird. Insbesondere vorteilhaft wirkt dabei auch die Melasse, die neben der Nährbodenbildung für die aeroben Bakterien wegen ihrer Klebrigkeit dafür sorgt, dass das Aufbereitungsmittel an den Feststoffen haften bleibt und nicht ohne weiteres entfernt werden kann. Auf diese Weise wird gewährleistet, dass das aerobe Bakterienwachstum homogen verteilt die Kompostierung vorantreibt.

## Patentansprüche

1. Verwendung eines neben Wasser Saponin oder ein Saponin in wirksamer Form enthaltenden Produkts als Aufbereitungsmittel für Abfallprodukte.

2. Verwendung nach Anspruch 1, dadurch gekennzeichnet, dass das Aufbereitungsmittel Reinsaponin in einer Menge von 10 bis 20 ppm im Wasser enthält.

3. Verwendung nach Anspruch 1 und/oder 2, dadurch gekennzeichnet, dass das Aufberitungsmittel einen Bakteriennährstoff enthält.

4. Verwendung nach Anspruch 3, dadurch gekennzeichnet, dass das Aufbereitungsmittel als Bakteriennährstoff Melasse enthält.

5. Verwendung nach einem oder mehreren der Ansprüche 1 bis 4, dadurch gekennzeichnet, dass das Aufbereitungsmittel ein wasserlösliches Hydrokolloid enthält.

6. Verwendung nach einem oder mehreren der Ansprüche 1 bis 5, dadurch gekennzeichnet, dass das Aufbereitungsmittel ein Konservierungsmittel enthält.

7. Verwendung nach Anspruch 6, dadurch gekennzeichnet, dass das Aufbereitungsmittel als Konservierungsmittel Sorbinsäure enthält.

8. Verwendung nach einem oder mehreren der Ansprüche 1 bis 7, dadurch gekennzeichnet, dass das Aufbereitungsmittel die folgende Zusammensetzung aufweist:

6 bis 20 Gew.-% Saponinprodukt mit einer Konzentration von 20 bis 30 Gew.-% Reinsaponin  
40 bis 50 Gew.-% Melasse  
1 bis 6 Gew.-% wasserlösliches Hydrokolloid  
0,1 bis 6 Gew.-% Konservierungsmittel  
Rest Wasser

9. Verwendung nach Anspruch 8, dadurch gekennzeichnet, dass das Aufbereitungsmittel in einer wässrigen Verdünnung von 3 bis 55 pm vorliegt.

10. Verwendung nach einem der Ansprüche 1 bis 9 für die Aufbereitung von Flüssigmist.

11. Verwendung nach einem der Ansprüche 1 bis 9 für die Kompostierung.

12. Verwendung nach einem der Ansprüche 1 bis 9 für die Aufbereitung von Abwasser.

13. Verwendung nach einem der Ansprüche 1 bis 9 für die Aufbereitung von Geflügelkot.

14. Verwendung nach einem der Ansprüche 1 bis 9 für die Aufbereitung von Nährstoffen enthaltenden landwirtschaftlichen Nebenprodukten.

## Claims

1. Use of a product containing, in addition to water, saponin or a saponin in active form, as a treatment agent for waste and effluent.

2. Use according to claim 1, characterised in that the treatment agent contains pure sapolin in an amount of 10 to 20 ppm in water.

3. Use according to claim 1 and/or 2, characterised in that the treatment agent contains a bacterial nutrient.

4. Use according to claim 3, characterised in that the treatment agent contains molasses as bacterial nutrient.

5. Use according to one or more of claims 1 to 4, characterised in that the treatment agent contains water-soluble hydrocolloid.

6. Use according to one or more of claims 1 to 5, characterised in that the treatment agent contains a preservative.

7. Use according to claim 6, characterised in that the treatment agent contains sorbic acid as preservative.

8. Use according to one or more of claims 1 to 7, characterised in that the treatment agent has the following composition:

6 to 20% by wt. of a saponin product with a concentration of  
20 to 30% by wt. pure saponin,  
40 to 50% by wt. of molasses,  
1 to 6% by wt. of water-soluble hydrocolloid,  
0.1 to 6% by wt. of preservative, and remainder water.

9. Use according to claim 8, characterised in that the treatment agent is present in an aqueous dilution of 3 to 55 ppm.

10. Use according to one of claims 1 to 9 for the treatment of liquid manure.

11. Use according to one of claims 1 to 9 for composting.

12. Use according to one of claims 1 to 9 for the treatment of waste water and effluent.

13. Use according to one of claims 1 to 9 for the treatment of poultry droppings.

14. Use according to one of claims 1 to 9 for the treatment of agricultural byproducts containing nutrients.

## Revendications

1. Utilisation comme agent de traitement des déchets, d'un produit contenant en dehors de l'eau de la saponine ou une saponine sous forme active.

2. Utilisation selon la revendication 1, caractérisée en ce que l'agent de traitement contient de la saponine pure dans l'eau en proportion de 10 à 20 ppm.

3. Utilisation selon la revendication 1 et/ou la revendication 2, caractérisée en ce que l'agent de traitement contient un aliment pour bactéries.

4. Utilisation selon la revendication 3, caractérisée en ce que l'agent de traitement contient de la melasse comme aliment de bactéries.

5. Utilisation selon l'une ou plusieurs des revendications 1 à 4, caractérisée en ce que l'agent de traitement contient un hydrocolloïde soluble dans l'eau.

6. Utilisation selon l'une ou plusieurs des revendications 1 à 5, caractérisée en ce que l'agent de traitement contient un agent de conservation.

7. Utilisation selon la revendication 6, caractérisée en ce que l'agent de traitement contient de l'acide sorbique comme agent de conservation.

8. Utilisation selon l'une ou plusieurs des revendications 1 à 7, caractérisée en ce que l'agent de traitement présente la formule suivante:

6 à 20% en poids de composant de saponine avec une concentration de 20 à 30% en poids de saponine pure,

40 à 50% en poids de mélasse,

1 à 6% en poids d'hydrocolloïde soluble dans l'eau,

0,1 à 6% en poids d'agent de conservation, le reste en eau.

9. Utilisation selon la revendication 8, caractérisée en ce que l'agent de traitement se présente en dilution aqueuse de 3 à 55 ppm.

10. Utilisation selon l'une des revendications 1 à 9 pour le traitement de fumier liquid.

11. Utilisation selon l'une des revendications 1 à 9 pour le traitement de compostages.

12. Utilisation selon l'une des revendications 1 à 9 pour le traitement des eaux usées.

13. Utilisation selon l'une des revendications 1 à 9 pour le traitement de fange de volaille.

14. Utilisation selon l'une des revendications 1 à 9 pour le traitement de sous-produits agricoles contenant des matières nutritives.